Europäisches Patentamt

European Patent Office

Office européen des brevets

(19)

(11) **EP 1 290 997 A1**

(12) **EUROPEAN PATENT APPLICATION**

(43) Date of publication:
**12.03.2003 Bulletin 2003/11**

(51) Int Cl.[7]: **A61K 6/083**

(21) Application number: **01121229.7**

(22) Date of filing: **05.09.2001**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI**<br><br>(71) Applicant: **Degussa AG**<br>**40474 Düsseldorf (DE)** | (72) Inventors:<br>• **Meyer, Dr. Jürgen**<br>**63811 Stockstadt (DE)**<br>• **El Moussaoui, Aziz**<br>**63505 Langenselbold (DE)**<br>• **Scholz, Dr. Mario**<br>**63584 Gründau (DE)**<br>• **Götz, Wolfgang**<br>**61130 Nidderau-Ostheim (DE)** |

(54) **Homogeneous microfilled dental composite material and method of preparation**

(57)    A homogeneous microfilled dental composite material comprised of functionalized silicas, characterised by functional groups fixed on the surface, the groups being 3-methacryloxypropylsilyl and/or glycidyloxypropylsilyl and/or functionalized, structurally modified silicas, characterized by functional groups fixed on the surface, the groups being 3-methacryloxypropylsilyl and/or glycidyloxypropylsilyl, with the following physico-chemical characteristic data:

| | | |
|---|---|---|
| BET surface area | m²/g | 20-380 |
| Tamped density | g/l | 50-600 |
| pH | | 3-10 |
| Carbon content | % | 0.1-15 |
| DBP number | % | <200 |

EP 1 290 997 A1

**Description**

BACKGROUND OF THE INVENTION

This invention relates to a homogeneous microfill composite material for use in dental restorations and for layering over microhybrids.

Microfill composite materials are generally produced by mixing finely divided silica with a polymerizable monomer, usually an acrylate or methacrylate-based resin, heat polymerizing the mixture in bulk, and pulverizing the mixture down to the desired agglomerate size to give a filler material comprised of splintered polymerized particles. This filler material is then mixed with a polymerizable monomer, again typically an acrylate or methacrylate-based resin, and an additional filler material, such as a colloidal silica. Thus, there are typically two polymerization steps, with the first being referred to as prepolymerization.

For example, U.S. Pat. No. 4,781,940 describes a process for producing a dental composite material using a prepolymerization step. A slurry of silica in a polymerizable monomer/solvent solution is prepared, followed by evaporating the solvent by heating at atmospheric pressure. The monomer coated silica particles are individualized by sieving, then polymerized by heating, and again sieved. No pulverization step is required. This filler material is then mixed with an additional filler and a resin, which may have the same monomers as those used in the monomer/solvent solution.

The composite material resulting from processes such as that of U.S. Pat. No. 4,781,940 is heterogeneous as a result of the prepolymerization step. Heterogeneous dental fillers are hydrolytically unstable and suffer from catastrophic marginal failures when used in dental restorations, in part, as a result of separation along the prepolymerized particle-resin matrix interface caused by percolation of aqueous fluids. Thus, it is desirable to develop a microfill that does not involve prepolymerization of the silica particles.

U.S. Pat. No. 4,389,497 is directed to a filler material which may or may not include a prepolymerization step, and therefore, may be heterogeneous or homogeneous. The inorganic filler material used is in the form of agglomerates of silicic acid, which can be produced with or without a binding agent. For example, the agglomerates could be formed by premixing silicic acid with a water glass solution, a boric acid solution, or an alcoholic aluminum alcoholate solution. The agglomerated material is then adjusted to the desired size by milling and screening. A further manner of production involves premixing the silicic acid with organo-silicon compounds preferably containing a polymerizable residue and a polymerization catalyst if necessary, followed by heat polymerization. The organic constituents are then burned and the mixture is brought up to more than 600 °C. Agglomerate reduction to the desired size is then achieved by milling and screening, subsequent to the heating step.

In addition to the homogeneous composites disclosed in U.S. Pat. No. 4,389,497, there are other homogeneous composites that have been offered commercially. These homogeneous composites have proved unsuccessful in that they are difficult to prepare reproducibly and the theological properties are difficult to control. This is due to difficulty in controlling the agglomeration of silica particles. Thus, it is desirable to develop a process in which the agglomeration can be controlled to prepare reproducibly a homogeneous microfill composite material.

The US patent 6,020,395 describes a homogeneous microfilled dental composite material, which contains as a filler the fumed silica Aerosil OX50, which is coated with gamma-methacryloxypropyltrimethoxysilane, milled and heated to a temperature range of 800-1000 °C.

During this calcination the silane is burned to carbondioxid and water and silica. The silica such formed has tendency to associate with itself to form siloxane units.

This fumed silica is then treated with an organic silane i.e. gamma-methacryloxypropyltrimethoxysilane, sieved to produce an avarage aggregate size of about 10 micrometer and then compounted in paste.

The present invention provides a hydrolytically stable, homogeneous microfilled dental composite material comprised of a polymerizable monomer mixed with silica particles for use in dental restorations, and a method for making the same, such that the material is easy to prepare reproducibly and the rheological properties of the material are controllable.

Subject of the invention is a dental composite material comprising functionalized silicas, characterised by functional groups fixed on the surface, the groups being 3-methacryloxypropylsilyl and/or glycidyloxypropylsilyl and/or functionalized, structurally modified silicas, characterized by functional groups fixed on the surface, the groups being 3-methacryloxypropylsilyl and/or glycidyloxypropylsilyl, with the following physico-chemical characteristic data:

| BET surface area | $m^2/g$ | 20-380 |
|---|---|---|
| Tamped density | g/l | 50-600 |
| pH | | 3-10 |
| Carbon content | % | 0.1-15 |

(continued)

| DBP number | % | <200 |
|---|---|---|

The functionalized silicas are produced by intensive mixing in a suitable mixing vessel first with water or dilute acid and then with a surface modification reagent or spraying mixture of several surface modification reagents. The components are optionally re-mixed for 15 to 30 minutes and heat-treated at a temperature of 100 to 400 °C over a period of 1 to 6 h.

A silica prepared pyrogenically by the route of flame of $SiCl_4$ can preferably be employed as the silica. Such pyrogenic silicas are known from Ullmanns Encyclopaedia of Industrial Chemistry, 4th edition, volume 21, page 464 (1982).

In a preferred embodyment of the invention, a pyrogenic silica with a surface area of approximately 200 $m^2$/g can be reacted (Aerosil 200).

Another preferred embodyment of the invention can be the use of the pyrogenic silica Aerosil OX50 (see table 1).

Monomeric substances, such as 3-methacryloxypropyltrialkoxysilane and/or glycidyloxypropyltrialkoxysilane, wherin alkoxy can be methoxy, ethoxy and/or propoxy can be employed as the surface modification reagent.

The amount of silane can be metered in with respect to the silica such that no excess results. The excess silane can optionally be removed during the heat treatment.

Table 1 provides the pyrogenic silicas, which can be used for producing functionalized and/or as for functionalized structurally modified silicas according to our invention.

These pyrogenic or fumed silicas can show a BET-surface from 35 to 410 $m^2$/g.

In a preferred subject of the invention the starting fumed silica can be elected from the group of silicas showing a BET-surface of 90 ± 15 and/or 130 ± 25 and/or 150 ± 15 and/or 200 ± 25 and/or 300 ± 30 and/or 380 ± 30 and/or 50 ± 15 and/or 200 ± 50 $m^2$/g.

In a specific preferred subject of the invention the starting fumed silica can be elected from a group showing a BET-surface of 50 ± 15 and/or 200 ± 25 $m^2$/g.

Table 1

| Physico-chemical data of AEROSIL | | | | | | | | | |
|---|---|---|---|---|---|---|---|---|---|
| **Test method** | | **AEROSIL 90** | **AEROSIL 130** | **AEROSIL 150** | **AEROSIL 200** | **AEROSIL 300** | **AEROSIL 380** | **AEROSIL OX 50** | **AEROSIL TT 600** |
| **Behaviour towards water** | hydrophilic | | | | | | | | |
| **Appearance** | loose white powder | | | | | | | | |
| **BET surface area**[1] | $m^2/g$ | 90±15 | 130±25 | 150±15 | 200±25 | 300±30 | 380±30 | 50±15 | 200±50 |
| **Average primary particle size** | nm | 20 | 16 | 14 | 12 | 7 | 7 | 40 | 40 |
| **Tamped density** approx. values[2] | g/l | 80 | 50 | 50 | 50 | 50 | 50 | 130 | 60 |
| compacted goods (added "V") | g/l | 120 | 120 | 120 | 120 | 120 | 120 | | |
| VV goods (added "VV")[12] | g/l | | | 50/75 | 50/75 | 50/75 | | | |
| | g/l | | | | 120 | 120 | | | |
| Loss on drying[3] (2 hours at 105 °C) on leaving supply works | % | <1.0 | <1.5 | <0.5[9] | <1.5 | <1.5 | <2.0 | <1.5 | <2.5 |

1) in accordance with DIN 66131

2) in accordance with DIN ISO 787/XI, JIS K 5101/18 (not sieved)

3) in accordance with DIN ISO 787/II, ASTM D 280. JIS K 5101/21

9) special packaging protecting against moisture

12) VV goods are currently supplied only from the Rheinfelden works

Table 1   (continued)

| Physico-chemical data of AEROSIL | | | | | | | | | |
|---|---|---|---|---|---|---|---|---|---|
| **Test method** | | **AEROSIL 90** | **AEROSIL 130** | **AEROSIL 150** | **AEROSIL 200** | **AEROSIL 300** | **AEROSIL 380** | **AEROSIL OX 50** | **AEROSIL TT 600** |
| Loss on ignition[4] [7] (2 hours at 1000°C) | % | <1 | <1 | <1 | <1 | <2 | <2.5 | <1 | <2.5 |
| pH[5] | | 3.7-4.7 | 3.7-4.7 | 3.7-4.7 | 3.7-4.7 | 3.7-4.7 | 3.7-4.7 | 3.8-4.8 | 3.6-4.5 |
| $SiO_2$[8] | % | >99.8 | >99.8 | >99.8 | >99.8 | >99.8 | >99.8 | >99.8 | >99.8 |
| $Al_2O_3$[8] | % | <0.05 | <0.05 | <0.05 | <0.05 | <0.05 | <0.05 | <0.08 | <0.05 |
| $Fe_2O_3$[8] | % | <0.003 | <0.003 | <0.003 | <0.003 | <0.003 | <0.003 | <0.01 | <0.003 |
| $TiO_2$[8] | % | <0.03 | <0.03 | <0.03 | <0.03 | <0.03 | <0.03 | <0.03 | <0.03 |
| HCl[8] [10] | % | <0.025 | <0.025 | <0.025 | <0.025 | <0.025 | <0.025 | <0.025 | <0.025 |
| Sieve residue[8] (Mocker method, 45 µm) | % | <0.05 | <0.05 | <0.05 | <0.05 | <0.05 | <0.05 | <0.2 | 0.05 |
| Drum size (net) [11] | kg | 10 | 10 | 10 | 10 | 10 | 10 | 10 | 10 |

4) in accordance with DIN 55921, ASTM D 1208, JIS K 5101/23

5) in accordance with DIN ISO 787/IX, ASTM D 1208, JIS K 5101/24 6) in accordance with DIN ISO 787/XVIII, JIS K 5101/20

7) based on the substance dried for 2 hours at 105 °C

8) based on the substance ignited for 2 hours at 1000 °C

10) HCl content is a constituent of the loss on ignition

11) v goods are supplied in sacks of 20 kg

The functionalized, structurally modified silicas can be produced in that a silica is sprayed optionally first with water or dilute acid and then with a surface modification reagent or a mixture of several surface modification reagents in a suitable mixing vessel, with intensive mixing, the components are optionally re-mixed for 15 to 30 minutes and optionally heat-treated optionally under inert gas, i.e. nitrogen at a temperature of 100 to 400 °C over a period of optionally 1 to 6 h, and the functionalized silica is then destructured/compacted by mechanical effects and optionally re-ground in a mill.

According to the invention, a ball mill, for example, can be employed for the destructuring. The re-grinding can be carried out, for example, by means of an air jet mill or pinned disc mill.

A homogeneous resin mixture consisting of a polymerizable organic acrylic monomer is prepared for mixing with the filler, which is the silica according to the invention and optionally a second compound. The second compound can be treated glass.

Another subject of the invention is a method for preparing composite material comprising combining a resin consisting of a polymerisable a crylic monomer and a filler which is the silica according to the invention and optionally a second compound and optionally cured.

The polymerizable acrylate monomers that may be used in the initiator and/or accelerator pastes of the present invention are well known in the art. See, e.g. U.S. Pat. No. 4,383,826; U.S. Pat. No. 4,333,348; and U.S. Pat. No. 3,066,112, these patents being incorporated herein by reference. Thus, some non-limiting examples of the polymerizable acrylate monomers which may be used include acrylic acid, methacrylic acid; typical alkyl methacrylates (e.g. methyl methacrylate, butyl methacrylate); cycloaliphatic methacrylates (e.g. cyclohexyl methacrylate; iso-bornyl methacrylate); aryl methacrylates (phenyl methacrylate; benzyl methacrylate; bisphenol dimethacrylates, etc.); functional methacrylates (such as 2-hydroxyethyl methacrylate; 4-methacryloxyethoxybenzaldehyde; 4-methacryloxyethoxybenzoic acid); ethylene glycol dimethacrylate; diethylene glycol dimethacrylate; triethylene glycol dimethacrylate; tetraethylene glycol dimethacrylate; polyethylene glycol and polypropylene glycol dimethacrylates; ethoxylated and propoxylated bisphenol dimethacrylates; neopentyl glycol dimethacrylate; trimethylolpropane trimethacrylate; 1,6-hexanediol-, 1,10-decanediol and 1,4-cyclohexanediol dimethacrylates; pentaerythritol tetramethacrylate; 1,10-decamethylene dimethacrylate; 1H, 1H-pentadecafluoroctyl methacrylate; 2,2-bis[p-(2'-hydroxy-3-methacryloxypropoxy)phenyl]propane, i.e. bis-GMA; and various nonhydroxylated homologs of bis-GMA e.g. 2,2-bis[p-(2'-methacryloxyethoxy)phenyl] propane; various diurethane dimethacrylates (e.g. the diadduct of 2-hydroxyethyl methacrylate and trimethylhexamethylene diisocyanate), oligomeric urethanes with multifunctional methacrylate groups (e.g. urethane derivatives of bis-GMA and aliphatic and cycloaliphatic diisocyanates), and other prepolymer types of monomers (e.g. siloxane multifunctional methacrylates; polyfluorinated oligomeric multifunctional methacrylates, etc.) and the like. Mixtures of the above and other acrylate monomers or other copolymerizable monomers (such as any vinyl monomer capable of free radical polymerization, e.g. styrene, alpha.-methylstyrene, vinyl biphenyl, vinyl acetate, pentafluorostyrene, and similar olefinic monomers) also may be used in either or both of the paste components. However, bulky multifunctional acrylic monomers are preferred since they have relatively low exotherms and shrinkages on polymerization, and because of their crosslinking nature, yield materials with low water sorption, high strength and excellent dimensional stability. In addition such monomers are desirable because of their low volatility and minimum potential for tissue irritation.

For example, the following components (in parts by weight) may be mixed together to give a preferred homogeneous resin mix:

    60-90 parts Diurethane Dimethacrylate (Rohamere 6661-0, Huls America, Inc. Piscataway, N.J.),
    10-40 parts Triethyleneglycoldimethacrylate,
    0.4-1.0 parts 2-Hydroxy-4-methoxybenzophenone,
    0.07-0.4 parts Camphorquinone,
    0.1-1.0 parts p-octyl dimethylamino-benzoate.

This cured resin system is tough and highly cross-linked. The goal in selecting a resin system is to provide a resin with a refractive index similar to that of silica, such that they are a relatively good match from a visual and aesthetic standpoint.

The filler, which is the silica according to the invention and optionally a second compound. The second compound can be treated glass, is then dispersed in resin by mixing together until uniform, approximately 3-10 hours, such as by mixing in a planetary mixer. The components are mixed in the following amounts:

    45-60 wt. % Filler, which is the silica according to the invention and optionally a second compound. The second compound can be treated glass.

    40-55 wt. % Resin.

A deaeration step is then carried out for at least 10 minutes, by which excess air in the composite paste material is eliminated by bubbling the air out in a vacuum. Deaerating is advantageously done in an attenuated oxygen atmosphere.

The microfill of the present invention exhibits better physical properties than the currently marketed microfills. For example, substantially higher flexural strength and toughness are obtained with the present invention. This assures higher fracture resistance than a typical microfill. This high strength and fracture resistance help reduce gouging during finishing. Better hydrolytic stability from reduced water percolation at the filler-resin matrix interface, higher mechanical properties and reduced crack propagation even in feathered edges are expected due to the silane treatment and the absence of prepolymerized particles.

Together, these improvements are expected to lead to better long term clinical performance, with the problem of catastrophic marginal failure, as observed with heterogeneous microfills, considerably lessened. Furthermore, the process of the present invention allows for control of the rheological properties due to easier control of the agglomerate of fumed silica by agglomerate reduction, during the structure modification. Moreover, the homogeneous microfill of the present invention is simpler to prepare reproducibly than the heterogeneous and homogeneous microfills of the prior art. Esthetic concerns are met in that the present process results in a material that is highly translucent, polishable and can be brought rapidly to a high luster that is retained after brushing and chewing.

The homogeneous resin matrix of the present invention leads to higher mechanical properties, such as flexural strength and toughness, and reduced crack propagation, thus reducing the chance of catastrophic marginal failure in dental restorative applications, as well as reducing gouging during finishing. A further advantage of the present invention is that the composite material is highly polishable and can be brought rapidly to a high luster, which is essential for materials used in exterior tooth applications. Furthermore, this high luster is retained after tooth brushing and chewing. Thus, there is provided a highly polishable, hydrolytically stable, homogeneous microfilled dental composite material with excellent mechanical properties for use in dental restoration.

Examples

For the estimation of the chemical-physical datas the following methods are used:

**Tamped density (approximate value) based on DIN ISO 787/XI August 1983**

1. Fundamentals

The tamped density of powders depends heavily on density, particle shape, particle size and densification. It allows conclusions to be drawn on e.g. the required size of packaging and the regularity of deliveries.

The tamped density (previously tamping volume) is equal to the ratio between the mass and the volume of a powder after it has been tamped in the Tap-Pak volumeter under stipulated conditions. The tamped density is specified in $g/cm^3$ in compliance with DIN ISO 787/XI. However, we specify the value in g/l due to the very low tamped density of synthetic silicic acids. Dehydration, straining and repitition of the tamping process are all foregone.

2. Apparatus

Tap-Pak volumeter
Measuring cylinder, 250 ml
Laboratory balance (accuracy of reading 0.01 g)

3. Administration

$200 \pm 10$ ml of silicic acid are filled into the measuring cylinder of the Tap-Pak volumeter so that there are no cavities and the surface is horizontal. The mass of the filled-in sample is determined to a precision of 0.01 g. The measuring cylinder with the sample is inserted in the measuring cylinder holder of the Tap-Pak volumeter and tamped 1250 times. The volume of the tamped silicic acid is read off to a precision of 1 ml.

4. Evaluation

$$\text{tamped density (g/l} = \frac{\text{g weight portion x 100}}{\text{ml read volume}}$$

**pH value 4 % dispersion**

1. Fundamentals

Aerosil is a highly disperse silicic acid which is produced by the "high-temperature hydrolysis process" and thus has a very high grade of purity. The gaseous hydrogen chloride can be separated from the stream of $SiO_2$. The pH value is a measure for the degree of deacidification of Aerosil and indicates physical, chemical and application-related properties.

2. Reagents

Destilled or demineralised water, pH > 5,5
Methanol, p.a.
Buffer solution, pH 7,00, pH 4,66

3. Apparatus

Laboratory balance (accuracy of reading 0.1 g)
Beaker, 250 ml
Magnetic stirrer
Magnetic stick, length 4 cm
Combined pH electrode
pH meter
Dispensette, 100 ml

4. Instructions

The determination is based on DIN/ISO 787/IX:

Calibration: The meter is calibrated with the buffer solutions prior to the pH measurement. Calibration must be carried out only once if several measurements are taken consecutively.

4 g hydrophobic are made into a paste in a 250 ml beaker with 48 g (61 ml) Methanol. The suspension is diluted with 48 g (48 ml) water and stirred for five minutes with a magnetic stirrer (no. of revs approx. 1000 min$^{-1}$) whilst the pH electrode is immersed. After switching off the stirrer, the reading for the pH value is taken after one minute of retention. The result is displayed by a decimal figure.

**Determination of carbon in surface-modified silicas by the element determinator LECO CS 244**

1. Summary

The sample is heated up in a ceramic crucible under a flow of oxigen. The carbon in the sample is converted to carbon dioxide. The formed carbon dioxide is measured by infrared (IR) absorption. A quantification is obtained by calibration of the instrument with reference materials. The applied element determinator is a LECO CS 244.

### 2. Apparatus

- element determinator LECO CS 244
- control console with build in balance, weighting accuracy ± 0,001 g
- ceramic crucibles

### 3. Reagents

- oxygen, purity of 99,99 % (v/v)
- accelerators: Lecocell II and iron chips

### 4. Procedure

### 4.1. Sample preparation

About 200 mg of the test portion are weighed into a ceramic crucible. The sample is covered by about 1 g of Lecocell II accelerator and about 0,7 g of iron chip accelerator. The crucible is then covered with a perforated lid and put on the auto loader of the high frequency (HF) induction fumace.

### 4.2. Calibration

The calibration is carried out with reference materials whose carbon content is close to the exspected content in the test samples. The reference materials should match the chemical composition of the test samples.

### 4.3. Measurement

The measurement is performed with the following instrument settings:

| | |
|---|---|
| HF furnace power | maximum, about 1800 °C |
| rinsing time | 10 s |
| measuring time | 45 s |
| oxygen flow rate | 4 l/min |
| measuring channel | 1 |

The determination is made at least in duplicate.

### 4.4. Calculation

The carbon content is automatically calculated by the instrument.

**Loss on drying**

Contrary to DIN ISO 787/II we don't use 10 g but 1 g weighed portion.

About 1 g of the substance is weighed to a precision of 0,1 mg in a weighing bottle with a lid which has been dried in a drying furnace for 2 hours at 105 °C. The build-up of dust is to be carefully avoided.
Then the weighing bottle is covered with the lid and the loss in weight is determined after the weighing bottle have cooled down in the desiccator over silica gel.

Evaluation

$$\% \text{ Loss on drying at } 105 \text{ }^{\circ}\text{C} = \frac{M0 - M1}{M0} \times 100$$

M0 = weighed portion [g]
M1 = weight of dried sample [g]
The result is displayed by a decimal figure.
The BET-surface is estimated in accordance with DIN 66131.

**Loss on ignition**

**2 hours at 1000 °C**

**relating to the dried substance (2 hrs at 105 °C)**

Fundamentals

The loss on ignition is determined at 1000 °C at which the chemically bonded water escapes as well as the physically bonded water.

Apparatus

Porcelain crucible with crucible lid
Muffle furnace
Analytical balance (accuracy of reading 0.1 mg)
Dessicator

Administration

Contrary to DIN 55 921, 0.3 - 1 g of the non-predried substance is weighed to a precision of 0.1 mg in a porcelain crucible with a crucible lid which has been ignited beforehand. It is then ignited in a muffle furnace for 2 hours at 1000 °C. The build-up of dust is to be carefully avoided. It has proven favourable to place the weighed sample in the muffle furnace whilst it is still cold. Stronger air turbulences in the porcelain crucibles are avoided when the furnace is slowly heated. After reaching 1000 °C, the ignition is continued for 2 hours. Then the crucibles are covered with crucible lids and the loss in weight is determined after the crucibles have cooled down in the desiccator over silica gel.

Evaluation

As the loss on ignition refers to the sample dried for 2 hours at 105 °C, the following computational formula can be established:

$$\% \text{ Loss on ignition} = \frac{m_o \times \dfrac{100 - LD}{100} - m_2}{m_o \times \dfrac{100 - LD}{100}} \times 100$$

$m_o$ = weighed portion (g)
LD = loss on drying (%) according to specification 0300
$m_2$ = weight of ignited sample (g)
The result is displayed by a decimal figure.

DBP-number

Carrying out the determination

12.5 g of silica or silicate are placed in the kneader in the Brabender plastograph. Under constant mixing (rotational speed of the kneader blades: 125 rpm), dibutyl phtalate flows into the mixture at a rate of 4 ml/min. The incorporation is carried out without a requirement for power. Toward the end of the determination, the silica/DBP mixture develops a consistency resembling putty, which is indicated by a sharp increase in the power requirement followed and measured by means of a scale. At a scale deflection of 300 (total deflection: 1000, scale graduation = 0.2 mkp), the kneader as well as the DBP dosing mechanism are switched off by an electrical contact. The synchronous motor for the supply of DBP is coupled to a counter mechanism, so that the consumption of DBP in ml can be read off at the end point.

Calculation

$$\text{DBP absorption in \%} = \frac{\text{ml DBP 100 x 1,047}}{12.5}$$

Example 1 (comparison)

Aerosil 200 is mixed with 4 parts of water and 18 parts of 3-methacryloxypropyl-trimethoxysilane (for example DYNASILAN-MEMO) and the mixture is heat-treated at 140 °C under an inert gas. The silica obtained has the following properties:

| | |
|---|---|
| BET **[$m^2$/g]** | 138 |
| Tamped density **[g/l]** | 52 |
| pH | 4.6 |
| C content **[%]** | 5.7 |
| Loss on drying **[%]** | 0.8 |
| Loss on ignition **[%]** | 9.7 |
| DBP number **[%]** | 228 |

Example 2 (comparison)

Aerosil 200 is mixed with 3 parts water and 16 parts 3-glycidyloxypropyltrimethoxysilane (for example DYNASILAN-GLYMO) and the mixture is heat-treated at 140 °C under an inert gas. The silica obtained has the following properties:

| | |
|---|---|
| BET [$m^2$/g] | 165 |
| Tamped density [g/l] | 53 |
| pH | 4.9 |
| C content [%] | 5.5 |
| Loss on drying [%] | 1.5 |
| Loss on ignition [%] | 8.7 |
| DBP number [%] | 242 |

Example 3 (according to the invention)

Aerosil 200 is mixed with 4 parts water and 18 parts 3-methacryloxypropyl-trimethoxysilane and the mixture is heat-treated at 140 °C under an inert gas. The silanized silica is then compacted to approx. 250 g/l on a continuously operating vertical ball mill.
The silica obtained has the following properties:

| | |
|---|---|
| BET [m$^2$/g] | 138 |
| Tamped density [g/l] | 242 |
| pH | 4.6 |
| C content [%] | 5.7 |
| Loss on drying [%] | 0.6 |
| Loss on ignition [%] | 8.9 |
| DBP number [%] | 122 |

Example 4 (according to the invention)

Aerosil OX50 is mixed with 1,5 parts water and 10,5 parts 3-methacryloxypropyl-trimethoxysilane and the mixture is heat-treated at 140 °C under an inert gas. The silica obtained has the following properties:

| | |
|---|---|
| BET [m$^2$/g] | 39 |
| Tamped density [g/l] | 178 |
| pH | 5.3 |
| C content [%] | 2.2 |
| Loss on drying [%] | 1.0 |
| Loss on ignition [%] | 2.2 |
| DBP number [%] | 78 |

Example 5 (according to the invention)

The silanized silica from example 4 is then structure modified on a continuously operating vertical ball mill. The silica obtained has the following properties:

| | |
|---|---|
| BET [m$^2$/g] | 37 |
| Tamped density [g/l] | 481 |
| PH | 5.1 |
| C content [%] | 2.2 |
| Loss on drying [%] | 0.3 |
| Loss on ignition [%] | 2.6 |
| DBP number [%] | 56 |

In order to avoid agglomeration in the dental composite the structure modified silica can be milled.

Example 6 (comparison)

50 parts of a methacrylic resin mixture and 20 parts fumed silica according to example 1 get mixed with a Speed Mixer. After complete incorporation of the silica 30 parts of treated glass are added into the mixture. The resulting formulation shows a very plastic behaviour, it was not spreadable and the viscosity was out of the rheometers measuring range.

Example 7 (according to the invention)

50 parts of a methacrylic resin mixture and 20 parts of a structure modified fumed silica (example 3) get mixed with a Speed Mixer. After complete incorporation of the silica 30 parts of treated glass are added into the mixture. The resulting formulation shows a free flowing easy spreading behaviour with a viscosity of 233 Pas. The yield point was about 377 Pa.

The rheological properties were determined with a rheometer based on cone/plate measuring system at a temperature of 23 °C .

Example 8 (according to invention)

50 parts of a methacrylic resin mixture and 20 parts fumed silica according to example 4 get mixed with a Speed Mixer. After complete incorporation of the silica 30 parts of treated glass are added into the mixture. The resulting formulation shows a pasty and spreadable behaviour. The viscosity was 1320 Pas. The yield point was about 542 Pa.

Example 9 (according to the invention)

50 parts of a methacrylic resin mixture and 20 parts of a structure modified fumed silica according to example 5 get mixed with a Speed Mixer. After complete incorporation of the silica 30 parts of treated glass are added into the mixture. The resulting formulation shows a free flowing and outstanding easy spreading behaviour with a viscosity of 22 Pas. The yield was about 10 Pa.

The rheological properties were determined with a rheometer based on cone/plate measuring system at a temperature of 23 °C.

Table 2

| Characterized of uncured preparations | | |
|---|---|---|
| | viscosity D=10 s$^{-1}$ [Pa*s] | yield point [Pa] |
| comparison example 6 | >10000 | >10000 |
| example 7 | 233 | 377 |
| comparison example 8 | 1320 | 542 |
| example 9 | 22 | 10 |

**Claims**

1. Dental composite material comprising functionalized silicas, **characterised by** functional groups fixed on the surface, the groups being 3-methacryloxypropylsilyl and/or glycidyloxypropylsilyl and/or functionalized, structurally modified silicas, **characterized by** functional groups fixed on the surface, the groups being 3-methacryloxypropylsilyl and/or glycidyloxypropylsilyl, with the following physico-chemical characteristic data:

| BET surface area | m$^2$/g | 20-380 |
|---|---|---|
| Tamped density | g/l | 50-600 |
| pH | | 3-10 |
| Carbon content | % | 0.1-15 |
| DBP number | % | <200 |

2. A method for preparing dental composite material according to claim 1:

   - combining a resin consisting of a polymerizable monomer and filler, which is the silica according to the invention and optionally a second compound and optionally cured.

3. A method for preparing dental composite material comprising combining resin consisting of 60-90 parts by weight of Diurethane Dimethacrylate, 10-40 parts by weight Triethyleneglycoldimethacrylate, 0.4-1.0 part by weight 2-Hydroxy-4-methoxybenzophenone, 0.07-0.4 part by weight Camphorquinone, and 0.1-1.0 part by weight p-octyl dimethylamino-benzoate with the silane-treated fumed silica and deaerating the composite material in an oxygen atmosphere.

4. A method of restoring a tooth comprising the steps of:

    - preparing the tooth for restoration; and

    - applying to the prepared tooth a dental restorative composite according to claim 1.

EP 1 290 997 A1

## PARTIAL EUROPEAN SEARCH REPORT

**European Patent Office**

which under Rule 45 of the European Patent Convention shall be considered, for the purposes of subsequent proceedings, as the European search report

Application Number
EP 01 12 1229

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | US 6 020 395 A (ANGELETAKIS CHRISTOS) 1 February 2000 (2000-02-01) * the whole document * | 1-4 | A61K6/083 |
| A | EP 0 315 186 A (DEN MAT CORP) 10 May 1989 (1989-05-10) * table 1 * | 1-4 | |
| A | EP 0 368 657 A (KURARAY CO) 16 May 1990 (1990-05-16) * claims; examples * | 1-4 | |
| A | US 4 297 266 A (IBSEN ROBERT L ET AL) 27 October 1981 (1981-10-27) * column 2, line 19 - line 60 * | 1-4 | |
| A,D | US 4 781 940 A (DENTON JR ROBERT K) 1 November 1988 (1988-11-01) | | |
| A,D | US 4 389 497 A (SCHMITT WERNER ET AL) 21 June 1983 (1983-06-21) | | **TECHNICAL FIELDS SEARCHED (Int.Cl.7)** A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claim 4 is directed to a method of treatment of the human/animal body (Article 52(4) EPC), the search has been carried out and based on the alleged effects of the composition.

| Place of search | Date of completion of the search | Examiner |
|---|---|---|
| THE HAGUE | 30 January 2002 | Cousins-Van Steen, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

15

**ANNEX TO THE EUROPEAN SEARCH REPORT
ON EUROPEAN PATENT APPLICATION NO.**                     EP 01 12 1229

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2002

| Patent document cited in search report | | Publication date | Patent family member(s) | | Publication date |
|---|---|---|---|---|---|
| US 6020395 | A | 01-02-2000 | NONE | | |
| EP 0315186 | A | 10-05-1989 | US | 4859716 A | 22-08-1989 |
| | | | AT | 88083 T | 15-04-1993 |
| | | | AU | 2478988 A | 11-05-1989 |
| | | | CA | 1337368 A1 | 17-10-1995 |
| | | | DE | 3880258 D1 | 19-05-1993 |
| | | | DE | 3880258 T2 | 29-07-1993 |
| | | | EP | 0315186 A2 | 10-05-1989 |
| | | | ES | 2053682 T3 | 01-08-1994 |
| | | | JP | 2000101 A | 05-01-1990 |
| | | | JP | 2791064 B2 | 27-08-1998 |
| EP 0368657 | A | 16-05-1990 | JP | 1940798 C | 09-06-1995 |
| | | | JP | 2134307 A | 23-05-1990 |
| | | | JP | 6067816 B | 31-08-1994 |
| | | | CA | 2002017 A1 | 11-05-1990 |
| | | | DE | 68908087 D1 | 09-09-1993 |
| | | | DE | 68908087 T2 | 18-11-1993 |
| | | | EP | 0368657 A2 | 16-05-1990 |
| | | | US | 5192815 A | 09-03-1993 |
| US 4297266 | A | 27-10-1981 | AT | 15594 T | 15-10-1985 |
| | | | AU | 528332 B2 | 21-04-1983 |
| | | | AU | 6921681 A | 31-08-1981 |
| | | | BR | 8106608 A | 22-12-1981 |
| | | | DE | 3172282 D1 | 24-10-1985 |
| | | | EP | 0045793 A1 | 17-02-1982 |
| | | | JP | 2044282 B | 03-10-1990 |
| | | | JP | 57500150 T | 28-01-1982 |
| | | | WO | 8102254 A1 | 20-08-1981 |
| US 4781940 | A | 01-11-1988 | NONE | | |
| US 4389497 | A | 21-06-1983 | DE | 3065482 D1 | 08-12-1983 |
| | | | WO | 8101366 A1 | 28-05-1981 |
| | | | EP | 0040232 A1 | 25-11-1981 |

EPO FORM P0459

For more details about this annex : see Official Journal of the European Patent Office, No. 12/82